(19) Europäisches Patentamt
European Patent Office
Office européen des brevets

(11) Veröffentlichungsnummer: **0 654 662 A1**

# (12) EUROPÄISCHE PATENTANMELDUNG

(21) Anmeldenummer: **94113358.9**

(51) Int. Cl.[6]: **G01N 21/64**

(22) Anmeldetag: **26.08.94**

(30) Priorität: **08.10.93 DE 4334327**

(43) Veröffentlichungstag der Anmeldung:
**24.05.95 Patentblatt 95/21**

(84) Benannte Vertragsstaaten:
**AT BE CH DE FR GB LI NL**

(71) Anmelder: **Forschungszentrum Karlsruhe GmbH**
**Weberstrasse 5**
**D-76133 Karlsruhe (DE)**

(72) Erfinder: **Moss, David, Dr.**
**Ringgässle 3**
**D-79112 Freiburg (DE)**
Erfinder: **Merz, Daniela**
**Marxzellerstrasse 11**
**D-76199 Karlsruhe (DE)**

(74) Vertreter: **Gottlob, Peter**
**Forschungszentrum Karlsruhe GmbH**
**Stabsabteilung**
**Patente und Lizenzen**
**Weberstrasse 5**
**D-76133 Karlsruhe (DE)**

(54) **Messgerät zum Nachweis von Photosystem-II-Herbiziden.**

(57) Beschrieben wird ein Meßgerät zum Nachweis von Photosystem-II-Herbiziden in wäßrigen Lösungen. Das Meßgerät besteht aus einer Anregungslichtquelle (1), die kurzzeitig Licht einer Wellenlänge zwischen 400 und 700 nm aussendet, zum Beispiel eine Laserdiode (12), zwei mit einer Suspension einer chlorophyllhaltigen photosynthetischen Präparation gefüllten Küvetten (3,4;16), die zur Aufnahme einer Proben- und einer Referenzlösung bestimmt sind, zwei Photodetektoren (6,7;14), die jeweils ausschließlich das aus den Suspensionen emittierte Fluoreszenzlicht registieren, einer Einrichtung (8,10), in der die Differenz der Photodetektorsignale gebildet wird, zum Beispiel ein Kleincomputer und mindestens einer Reoxidationslichtquelle (9;13) in Form einer LED, die kurzzeitig Licht einer Wellenlänge zwischen 700 und 800 nm in die beiden Küvetten einstrahlt.

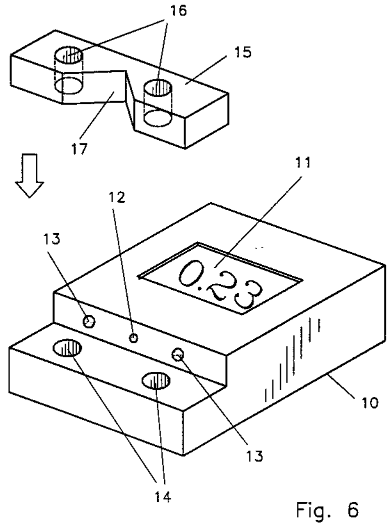


Fig. 6

EP 0 654 662 A1

Die Erfindung betrifft ein Meßgerät zum Nachweis von Photosystem-II-Herbiziden gemäß dem ersten Patentanspruch.

Zu den Photosystem-II-Herbiziden gehört eine Vielzahl strukturell völlig unterschiedlicher Verbindungen. Die wichtigsten Vertreter dieser Verbindungen sind Dichlorphenyldimethylharnstoff (DCMU, Diuron), Atrazin und Trimercaptotriazin (TMT).

Das Meßprinzip des Geräts beruht auf der Hemmung des photosynthetischen Elektronentransports in isolierten und stabilisierten chlorophyllhaltigen photosynthetischen Präparationen wie z. B. Choroplasten oder Thylakoiden. Der betroffene Abschnitt der photosynthetischen Elektronentransportkette läßt sich wie folgt darstellen:

$$H_2O \dashrightarrow Chl \xrightarrow{hv} Q_A \xrightarrow{Herbizid\ \Vert} \text{Photosystem-I-Chinone}$$

wobei jeder Pfeil einen Elektronentransfer, hv eingestrahltes Licht und Chl Chlorophyll bezeichnet. Wird dieses System an der bezeichneten Stelle durch ein Herbizid gehemmt, so führt eine Lichteinstrahlung zu einer schnellen Reduktion des Chinons $Q_A$. Ohne Herbizid verläuft diese Reduktion deutlich (ca. 100-fach) langsamer.

Wenn das Chinon $Q_A$ reduziert ist, kann die lichtgetriebene Reaktion Chl $\dashrightarrow$ $Q_A$ nicht mehr ablaufen; die überschüssige Lichtenergie wird vom Chlorophyllmolekül als Fluoreszenzlicht abgestrahlt. Die resultierende Erhöhung der Chlorophyllfluoreszenzintensität stellt die empfindlichste Methode dar, den Reduktionsgrad von $Q_A$ und damit die Konzentration der den Elektronenübergang hemmenden Herbizide zu messen.

Aus der Veröffentlichung "Algen-Fluoreszenz-Autometer, eine computergesteuerte Biotest-Meßapparatur" von F. Sayk und Ch. Schmidt in Z. Wasser- Abwasser-Forsch. **19**, 182-184 (1986) ist eine Meßeinheit zum Nachweis von Photosystem-II-Herbiziden bekannt, die auf dem beschriebenen Meßprinzip beruht. Diese Meßeinheit enthält als Anregungslichtquelle eine Weißlichtlampe und ein nachgeschaltetes optisches Filter, das nur für Anregungslicht einer Wellenlänge zwischen 400 und 550 nm durchlässig ist. Mit dem Anregungslicht wird jeweils eine von mehreren Proben- und Referenzküvetten bestrahlt. In den Strahlengang des Anregungslichts ist ein Photoverschluß integriert, der eine kurzzeitige (1/100 sec bis mehrere Sekunden) Belichtung der Küvette ermöglicht. Die Küvetten enthalten eine Suspension von Grünalgen, die mit Hilfe eines Magnetrührers in der Schwebe gehalten werden.

Unter dem Einfluß des Anregungslichts emittieren die Algen Fluoreszenzlicht, dessen Intensität bei Anwesenheit von Photosystem-II-Herbiziden ansteigt. Die Intensität des Fluoreszenzlichts wird nach Ausblendung des Anregungslichts mittels eines Kantenfilters bei einer Wellenlänge von 685 ± 5 nm mit Hilfe eines Photomultipiers gemessen. Sowohl die komplette Belichtungseinrichtung als auch der Photomultiplier sind auf einem exakt justierten Schlitten angebracht, der mittels Schrittmotoren an jede gewünschte Küvette herangefahren werden kann.

Für jede Küvette wird die Intensitätskurve des Fluoreszenzlichts in Abhängigkeit von der Zeit bestimmt und in einem Computer gespeichert. Probelösungen werden als herbizidbelastet angesehen, wenn ihre Meßwerte im Vergleich zu einer unbelasteten Referenzmessung eine Abweichung von 10 % ergeben.

Die bekannte Meßeinheit erscheint aus verschiedenen Gründen nicht befriedigend. Die Messung erfordert lebende Algenzellen, die permanent in einem angeschlossenen Fermenter gezüchtet werden müssen. Hierfür ist geschultes Personal notwendig. Chlorophyllfluoreszenzmessungen an lebenden Algenzellen erfassen nicht nur die Photosystem-II-Herbizide, sondern auch eine Vielzahl von anderen Substanzen, die nur indirekt die Photosynthese beeinflussen. Zudem wird angegeben, daß mit einer 90-minütigen Inkubationszeit gearbeitet wird; damit wird die Empfindlichkeit gegenüber diesen anderen Substanzen verstärkt. Die Meßeinheit ist durch den Einsatz des exakt zu justierenden Schlittens sehr kompliziert; darüberhinaus ist das Auswerteverfahren nicht optimal, wie sich aus der angegebenen Nachweisgrenze von 0,0325 mg Atrazin/l Probelösung ergibt.

Für die Bestimmung von Photosystem-II-Herbiziden durch Fluoreszenzmessungen ist das Cyanobakterium Synechococcus leopoliensis Komarek vorgeschlagen worden, das gegenüber diesen Herbiziden empfindlicher reagiert als Wildstämme und den Chloroplasten der höheren Pflanzen vergleichbar ist (K.-H. Pastrik, U. Kärst und R. D. Schmid, "Mutanten von Cyanobakterien mit erhöhter Empfindlichkeit gegenüber Herbiziden", Z. Wasser- Abwasser-Forsch. **24**, 12-15 (1991)).

Eine Meßanordnung, in der immobilisierte Cyanobakterien als chlorophyllhaltiges biologisches Material eingesetzt werden, ist von R. D. Schmid, A. Gebbert, R. Kindervater und P. Krämer in "Biosensoren zur

Analytik von Pflanzenschutzmitteln in Wasser", Z. Wasser- Abwasser-Forsch. **24**, 15-20 (1991) beschrieben. Die Meßanordnung erfordert eine Kultur von Cyanobakterien und den Einsatz von Hilfschemikalien (Kaliumhexacyanoferrat).

Der Erfindung liegt die Aufgabe zugrunde, ein Meßgerät zum Nachweis von Photosystem-II-Herbiziden, insbesondere von Triazin- und Phenylharnstoff-Herbiziden, in wäßrigen Lösungen vorzuschlagen, bei dem als Chlorophyllträger keine lebenden Zel-len und keine Hilfschemikalien notwendig sind. Das Meßgerät soll keine sich bewegenden Teile enthalten und so einfach aufbaubar sein, daß es sich auch für Feldmessungen eignet.

Die Aufgabe wird erfindungsgemäß durch das im ersten Patentanspruch beschriebene Meßgerät gelöst. Bevorzugte Ausgestaltungen des erfindungsgemäßen Meßgeräts sind in den Ansprüchen 2 und 3 angegeben. Anspruch 4 beschreibt die Präparation einer Thylakoidensuspension für den Einsatz im erfindungsgemäßen Meßgerät. Anspruch 5 gibt eine bevorzugte Präparation an.

Die Erfindung wird im folgenden anhand von Figuren erläutert.

Es zeigen

Fig. 1 ein Blockschaltbild des erfindungsgemäßen Meßgeräts;

Fig. 2 die Fluoreszenzintensitätskurven für belastete und unbelastete Proben;

Fig. 3 eine schematische Darstellung des Auswerteverfahrens;

Fig. 4 die Dosiswirkungskurve am Beispiel des Herbizids Terbutylazin;

Fig. 5 die Reoxidation bei Belichtung im Vergleich zur Dunkeladaption.

In Fig. 1 ist ein Blockschaltbild des erfindungsgemäßen Meßgeräts dargestellt. Als Anregungslichtquelle 1 wird vorzugsweise eine Laserdiode eingesetzt. Obwohl die zur Zeit kommerziell erhältlichen Laserdioden Licht mit einer Wellenlänge von 635 und 655 nm und damit am oberen Ende des optimalen Wellenlängenbereiches für Anregungslicht (400 bis 700 nm) abstrahlen, wird der konstruktive Aufbau des Meßgeräts durch Laserdioden wesentlich vereinfacht. Für Fälle, in denen ein kompaktes Meßgerät nicht unbedingt erforderlich ist, kann jedoch auch jede andere ausreichend intensive Lichtquelle eingesetzt werden, die Licht im angegebenen Wellenlängenbereich emittiert.

In der gezeigten Ausführungsform wird das Licht aus der Anregungslichtquelle auf einen Strahlteiler 2 geführt, von dem aus es in die beiden Küvetten 3 und 4 gelangt. Der Strahlteiler bewirkt eine absolut gleiche Beleuchtungsstärke beider Küvetten mit dem Anregungslicht. Die Küvetten müssen für die Messung mit chlorophyllhaltigem Material, insbesondere mit Chloroplasten oder Thylakoiden, gefüllt sein. In die erste Küvette 3 wird außerdem die zu untersuchende Probe, z. B. eine Trinkwasserprobe, eingefüllt. Die zweite Küvette 4 enthält unbelastetes Wasser als Referenz. Das chlorophyllhaltige Material ist in beiden Küvetten suspendiert.

An die Küvetten 3 und 4 schließen sich in dieser Ausführungsform optische Filter an, die verhindern, daß Anregungslicht in die beiden nachfolgenden Photodetektoren 6 und 7 gelangt. Die Photodetektoren 6 und 7 sind in der Weise relativ zu den beiden Küvetten 3 und 4 angeordnet, daß sie ausschließlich das in den Chloroplasten oder Thylakoiden der jeweils zugehörigen Küvette emittierte Fluoreszenzlicht registrieren. Mit Hilfe der beiden Photodetektoren 6, 7 wird jeweils eine Fluoreszenzintensität/Zeit-Kurve der Probe und der Referenz aufgenommen. Die Meßsignale werden einer Einrichtung 8, z. B. einem Computer, zugeführt, die die Differenz der beiden Kurven bildet und anzeigt.

In Fig. 2 sind beispielhaft mit Chloroplasten erhaltene Fluoreszenzintensität/Zeit-Kurven für eine belastete Probe und eine unbelastete Referenz angegeben. Man sieht, daß in unbelasteten Proben die Fluoreszenzintensität wesentlich später ansteigt.

Fig. 3 stellt schematisch das Auswerteverfahren dar. Die Anregungslichtquelle 1 bestrahlt die Küvette 3 mit einer belasteten Probe und die Küvette 4 mit der unbelasteten Referenzlösung. Beide Küvetten sind mit einer Choroplastensuspension gefüllt. Die jeweiligen Fluoreszenzintensität/Zeit-Kurven (a) und (b) werden durch die Photodetektoren (nicht dargestellt) registriert. Die Kurve (c) stellt die Differenz der beiden Fluoreszenzintensität/Zeit-Kurven (a) und (b) dar. Ihre Höhe ist proportional zu der gesamten Konzentration der Photosystem-II-Herbizide in der belasteten Probe.

Fig. 4 zeigt die Dosiswirkungskurve am Beispiel einer mit Terbutylazin belasteten Probe. Man sieht, daß mit dem erfindungsgemäßen Meßgerät die Konzentration von Terbutylazin bis in die Nähe von 1 ppb erfaßbar wird. Für das Herbizid Diuron ist eine Nachweisgrenze von 0,5 $\mu$g/l ermittelt worden.

Für den praktischen Betrieb des erfindungsgemäßen Meßgeräts ist es von entscheidender Bedeutung, daß mit der selben Probe nacheinander in kurzem zeitlichen Abstand mehrere Messungen durchgeführt werden können. Zum einen lassen sich dadurch Fehlmessungen schnell erkennen; zum andern wird die Empfindlichkeit des Meßgeräts deutlich verbessert, wenn aus möglichst vielen Fluoreszenzintensität/Zeit-Kurven eine Mittelwert-Differenzkurve gebildet wird; der statistische Fehler des Meßergebnisses läßt sich dadurch wesentlich vermindern.

Fig. 5 (untere Kurve) zeigt die Dunkeladaption der Chloroplasten, d. h. diejenige Zeit, die abgewartet werden muß, bevor eine erneute Messung eingeleitet werden kann. Man sieht, daß ohne zusätzliche Maßnahmen im Fall der Dunkeladaption mehrere Minuten verstreichen, bevor der Ausgangszustand wieder erreicht ist. In der Praxis muß aus Sicherheitsgründen noch eine zusätzliche Zeit von ebenfalls mehreren Minuten abgewartet werden, denn die Dunkeladaption ist von den äußeren Bedingungen abhängig; ist das System noch nicht vollständig reoxidiert, wird mit der nachfolgenden Messung ein falsches Ergebnis erzielt.

Dieser Nachteil wird beim erfindungsgemäßen Meßgerät dadurch beseitigt, daß nach der Aufnahme der Fluoreszenzintensität/Zeit-Kurven die Chloroplasten sowohl in der Proben- als auch in der Referenzküvette mit Reoxidationslicht einer Wellenlänge zwischen 700 und 800 nm bestrahlt werden. Dadurch wird das Chinon $Q_A$ praktisch sofort wieder in den oxidierten Zustand überführt. Die obere Kurve in Fig. 4 zeigt die Wirkung einer Belichtung mit Licht einer Wellenlänge von 715 nm. Unmittelbar nach der Bestrahlung mit dem Reoxidationslicht kann erneut ein Meßvorgang eingeleitet werden.

Im erfindungsgemäßen Meßgerät ist daher mindestens eine Reoxidationslichtquelle vorgesehen, mit deren Licht der Wellenlänge zwischen 700 und 800 nm die Chloroplasten oder Thylakoiden sowohl in der Proben- als auch in der Referenzküvette bestrahlt werden.

In Fig. 1 sind zwei Reoxidationslichtquellen 9 dargestellt, die jeweils eine Küvette bestrahlen. Als Reoxidationslichtquelle eignen sich insbesondere Leuchtdioden (Light-emitting diodes,LED's), vor allem solche, die ein Licht der Wellenlänge von 750 nm abstrahlen.

Fig. 6 zeigt eine besonders bevorzugte Ausführungsform des erfindungsgemäßen Meßgeräts. Dieses Meßgerät besteht aus einer Einheit 10, die als Einrichtung zur Differenzbildung einen Kleincomputer enthält und außerdem eine Anzeige 11 für das Meßergebnis, eine Laserdiode 12 (635 nm), zwei Reoxidationslichtquellen 13 in Form zweier LED's (750 nm) und zwei Photodioden 14 aufweist. Auf die Einheit 10 ist ein Aufsatz 15 aufsetzbar, in den zwei Küvetten 16 und ein Strahlteiler 17 integriert sind. Der Aufsatz 15 läßt sich in der Weise auf die Einheit 10 aufsetzen, daß das Licht der Laserdiode 12 über den Strahlteiler 17 beide Küvetten 16 bestrahlt. Das Fluoreszenzlicht läßt sich mit den Photodioden 14 (die das Anregungslicht nicht registrieren) detektieren. Die Signale der Photodioden werden mit Hilfe des Kleincomputers ausgewertet. Jede der beiden Küvetten 17 läßt sich nach der Messung mit einer Reoxidationslichtquelle 13 bestrahlen.

Eine wichtige Bedingung für den praktischen Betrieb des erfindungsgemaßen Meßgeräts besteht in der einfachen Präparation und der ausreichenden zeitlichen Beständigkeit der chlorophyllhaltigen photosynthetischen Präparation, insbesondere der Chloroplasten oder Thylakoiden. Eine Chloroplastensuspension kann gemäß dem in Anspruch 4 beschriebenen Verfahren hergestellt werden.

Die Erfindung wird im folgenden anhand von Durchführungsbeispielen näher erläutert.

Beispiel 1

Herstellung einer Chloroplasten-Suspension

Präparation aus Erbsenpflanzen

10 bis 13 Tage alte Erbsenpflanzen aus Erbsensaatgut Progress Nr. 9 wurden in 3 cm lange Stücke geschnitten und 40 g der Pflanzen in 250 ml eines halbgefrorenen wäßrigen Isolationspuffers homogenisiert. Der Isolationspuffer wies einen pH von 7,8 (KOH) auf und hatte die folgende Zusammensetzung:

| | |
|---|---|
| Saccharose | 340 mM |
| 4-(2-Hydroxyethyl)-Piperazin-1-ethan sulfonsäure (HEPES) | 2 mM |
| KCl | 0,4 mM |
| Ethylendiamintetraessigsäure (EDTA) | 0,04 mM |

Mit einer Ultrazentrifuge wurde die Mischung bei 24000 $min^{-1}$ innerhalb von weniger als 5 s homogenisiert. Anschließend wurde die Suspension durch 10 Lagen Mull in einen vorgekühlten 500 ml - Zentrifugenbecher filtriert und anschließend zentrifugiert. Die überstehende Lösung wurde dekantiert und Lösungsreste mit Watte entfernt. Der Chloroplasten-Rückstand wurde mit Watte in wenig gekühltem Isolationspuffer suspendiert. Die Watte wurde ausgespült und die Suspension mit 80 ml einer 10 mM $MgCl_2$-Lösung versetzt. Nach 15 s werden 80 ml des Isolationspuffers mit doppelter Konzentration hinzugefügt und nochmals zentrifugiert. Nach dem Dekantieren der überstehenden Lösung wurde der Rückstand wiederum mit Watte aufgenommen und in wenig wäßrigem Suspensionspuffer suspendiert. Der

Suspensionspuffer wies ebenfalls einen pH-Wert von 7,8 (KOH) auf und hatte die folgende Zusammensetzung:

| | |
|---|---|
| Sorbitol | 330 mM |
| HEPES | 50 mM |
| KCl | 10 mM |
| $MgCl_2$ | 1 mM |
| EDTA | 1 mM |
| $MnCl_2$ | 1 mM |

Die Suspension wurde mit 5 % Dimethylsulfoxid (DMSO) als Gefrierschutz versetzt. Alle Zentrifugationen wurden unter den folgenden Bedingungen durchgeführt:

| | |
|---|---|
| Temperatur: | 0 °C |
| max. Geschwindigkeit | 3000 g (30 s) |
| Beschleunigungszeit | 30 s |
| Abbremszeit | 30 s. |

Zur Bestimmung des Chloropyllgehalts wurden 50 µl der erhaltenen Suspension in 10 ml 80 %igem Aceton in einem handelsüblichen Zweistrahlphotometer gegen 80 %iges Aceton als Referenzlösung vermessen. Wird die erhaltene Absorptionsdifferenz mit $\triangle E$ bezeichnet, läßt sich daraus das Pipettiervolumen der Suspension errechnen, mit dem beide Küvetten gefüllt werden. Für eine vorgegebene Konzentration von 10 µg Chlorophyll/ml ergibt sich bei einem Extinktionskoeffizienten von $\epsilon$ = 34,5 $[(\mu g/\mu l)^{-1}\ cm^{-1}]$ ein Pipettiervolumen V [µl] von:

$$V\ [\mu l] = 34{,}5 \cdot 10/\triangle E \cdot 200$$

In jede Küvette wird das errechnete Pipettiervolumen der erhaltenen Suspension vorgelegt. Die Küvetten werden bei -30 °C aufbewahrt. Zur Messung wird zusätzlich in eine Küvette 500 µl der Probe und in die andere Küvette 500 µl destilliertes Wasser zugegeben.

Beispiel 2

Herstellung einer Chloroplasten-Suspension

Präparation aus Kopfsalat

Die äußeren, dunkelgrünen Blätter des Kopfsalats wurden zur Präparation gesäubert, abgetrocknet und in ca. 3 • 1 cm große Stücke geschnitten.

Die Herstellung der Chloroplasten-Suspension erfolgte analog zu Beispiel 1, wobei der Isolationspuffer zusätzlich 5 mM Ascorbinsäure enthielt.

Beispiel 3

Untersuchungen zur Lagerstabilität der Chloroplasten-Suspensionen nach Beispiel 1 und 2

Um die Lagerstabilität der nach Beispiel 1 und 2 erhaltenen Chloroplasten-Suspensionen zu überprüfen, wurden die Suspensionen bei verschiedenen Temperaturen gelagert und die Veränderung der Aktivität der Chloroplasten mittels einer Sauerstoffmessung überprüft. Die Temperaturen wurden anwendungsspezifisch ausgewählt (Umgebungstemperatur, Kühlschrank, Tiefkühlschrank).

In allen Fällen ergab sich ein einfach-exponentieller Abfall der Aktivität mit der Zeit. Für die Lagerung bei den verschiedenen Temperaturen wurden folgende Halbwertszeiten gefunden, wobei als Halbwertszeit diejenige Zeit bezeichnet wird, in der die Aktivität auf die Hälfte der ursprünglichen Aktivität abgefallen ist:

| | | |
|---|---|---|
| Lagerung | bei 27 °C<br>bei 4 °C<br>bei -20 °C | 127 Minuten<br>32 Stunden<br>21 Tage. |

Nachdem gezeigt werden konnte, daß sich die mögliche Lagerzeit von Chloroplasten-Suspensionen bei einer Lagertemperatur von -20 °C deutlich verlängert, wurde die Veränderung der Fluoreszenzkinetiken bei diesem "Alterungsprozeß" ermittelt. Dabei ergab sich, daß die bei -20 °C gelagerten Suspensionen noch einen Monat nach der Präparation ein praktisch unverändertes Fluoreszenzverhalten zeigten. Bei der in Fig. 6 gezeigten Ausführungsform kann daher der mit der Chloroplasten-Suspension befüllte Aufsatz im Tiefkühlschrank für mindestens einen Monat gelagert werden und zur Messung auf die Einheit 10 aufgesetzt werden. Wenn danach die Probe- und die Referenzlösung in die Küvetten 16 eingefüllt werden, ist das Meßgerät unmittelbar meßbereit. Das Meßgerät in der Ausführungsform nach Fig. 6 eignet sich daher insbesondere für Feldmessungen.

**Patentansprüche**

1. Meßgerät zum Nachweis von Photosystem-II-Herbiziden, insbesondere von Triazin- und Phenylharnstoff-Herbiziden, in wäßrigen Lösungen mit
   a) einer Anregungslichtquelle, die kurzzeitig Anregungslicht einer Wellenlänge zwischen 400 und 700 nm aussendet;
   b) einer ersten und einer zweiten Küvette, in die das Anregungslicht einfällt, die mit einer Suspension einer chlorophyllhaltigen photosynthetischen Präparation gefüllt sind, wobei die erste Küvette zur Aufnahme einer Probelösung und die zweite Küvette zur Aufnahme einer Referenzlösung bestimmt ist;
   c) einem ersten Photodetektor, der der ersten Küvette in der Weise nachgeschaltet ist, so daß er ausschließlich das Fluoreszenzlicht registriert, das von der Suspension der chlorophyllhaltigen photosynthetischen Präparation in der ersten Küvette emittiert wird;
   d) einem zweiten Photodetektor, der der zweiten Küvette in der Weise nachgeschaltet ist, so daß er ausschließlich das Fluoreszenzlicht registriert, das von der Suspension der chlorophyllhaltigen photosynthetischen Präparation in der zweiten Küvette emittiert wird,
   e) wobei sowohl der erste als auch der zweite Photodetektor ein Signal abgeben, dessen Intensität mit der Intensität des jeweils auftreffenden Fluoreszenzlichts korreliert;
   f) einer Einrichtung, mit der sich die Differenz der beiden Signale bestimmen läßt;
   g) mindestens einer Reoxidationslichtquelle, die kurzzeitig ein Licht einer Wellenlänge zwischen 700 und 800 nm aussendet, das sowohl in die erste als auch in die zweite Küvette einfällt, wobei die Reoxidationslichtquelle im Anschluß an die Anregungslichtquelle aktivierbar ist.

2. Meßgerät nach Anspruch 1 mit Thylakoiden als chlorophyllhaltiger photosynthetischer Präparation.

3. Meßgerät nach Anspruch 1 oder 2 mit einer Laserdiode als Anregungslichtquelle.

4. Meßgerät nach Anspruch 1, 2 oder 3 mit einer Leuchtdiode (Light-emitting diode, LED) als Reoxidationslichtquelle.

5. Verfahren zur Herstellung einer Suspension einer chlorophyllhaltigen photosynthetischen Präparation mit den Schritten:
   a) Homogenisierung von pflanzlichem Material in einem Isolationspuffer,
   b) Abtrennung der festen Bestandteile der entstehenden Suspension,
   c) Resuspension der festen Bestandteile der Suspension,
   d) Isolierung der Thylakoiden des pflanzlichen Materials durch Behandeln der Resuspension mit einer $MgCl_2$-Lösung,
   e) Zugabe von konzentriertem Isolationspuffer und Zentrifugation,
   f) Resuspension des Zentrifugationsrückstands in Suspensionspuffer.

6. Verfahren nach Anspruch 4 mit Kopfsalat oder Erbsenpflanzen als pflanzlichem Material.

Fig. 1

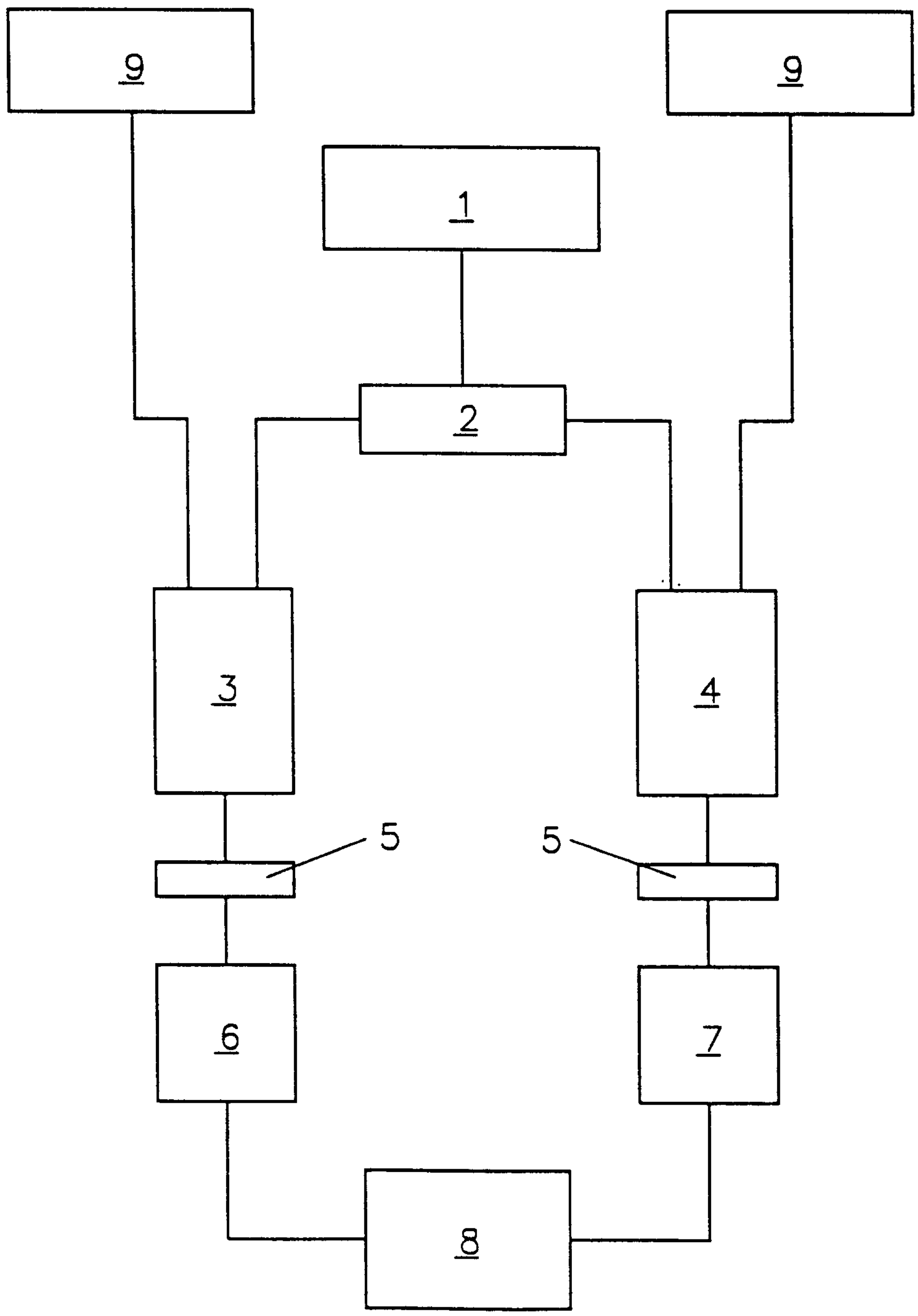

Fig. 2

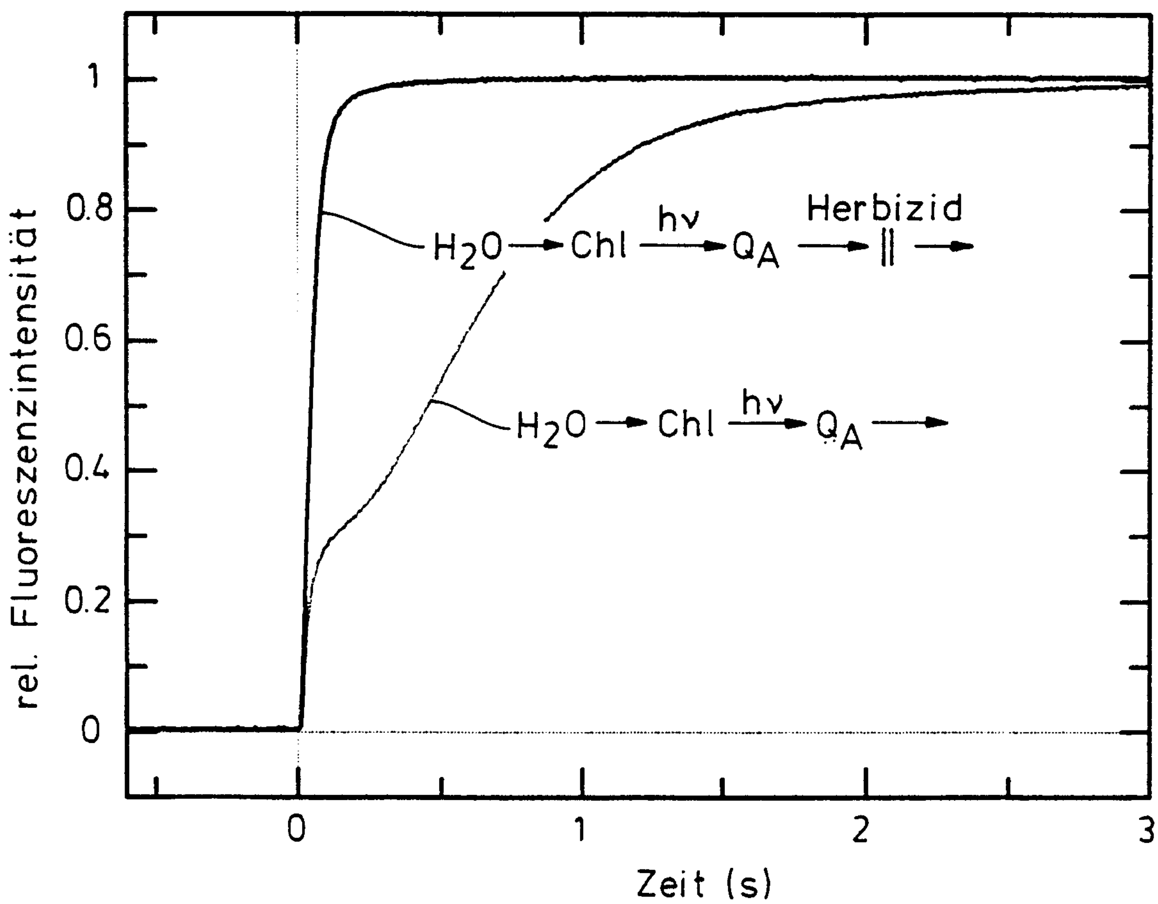
1
0.8
0.6
0.4
0.2
0
rel. Fluoreszenzintensität
0
1
2
3
Zeit (s)
H2O → Chl hν → QA → ‖ →
Herbizid
H2O → Chl hν → QA →

Fig. 3

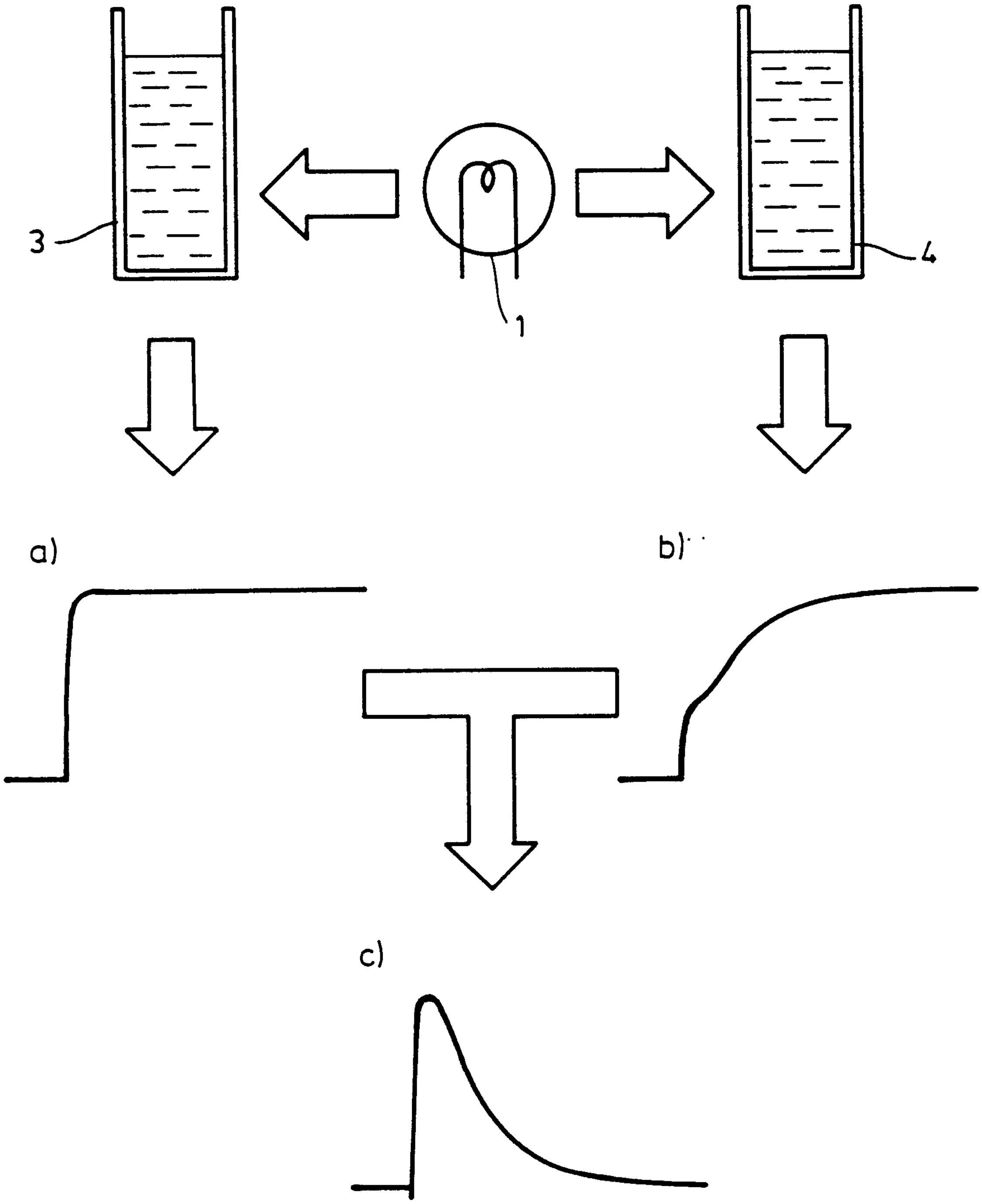

Fig. 4

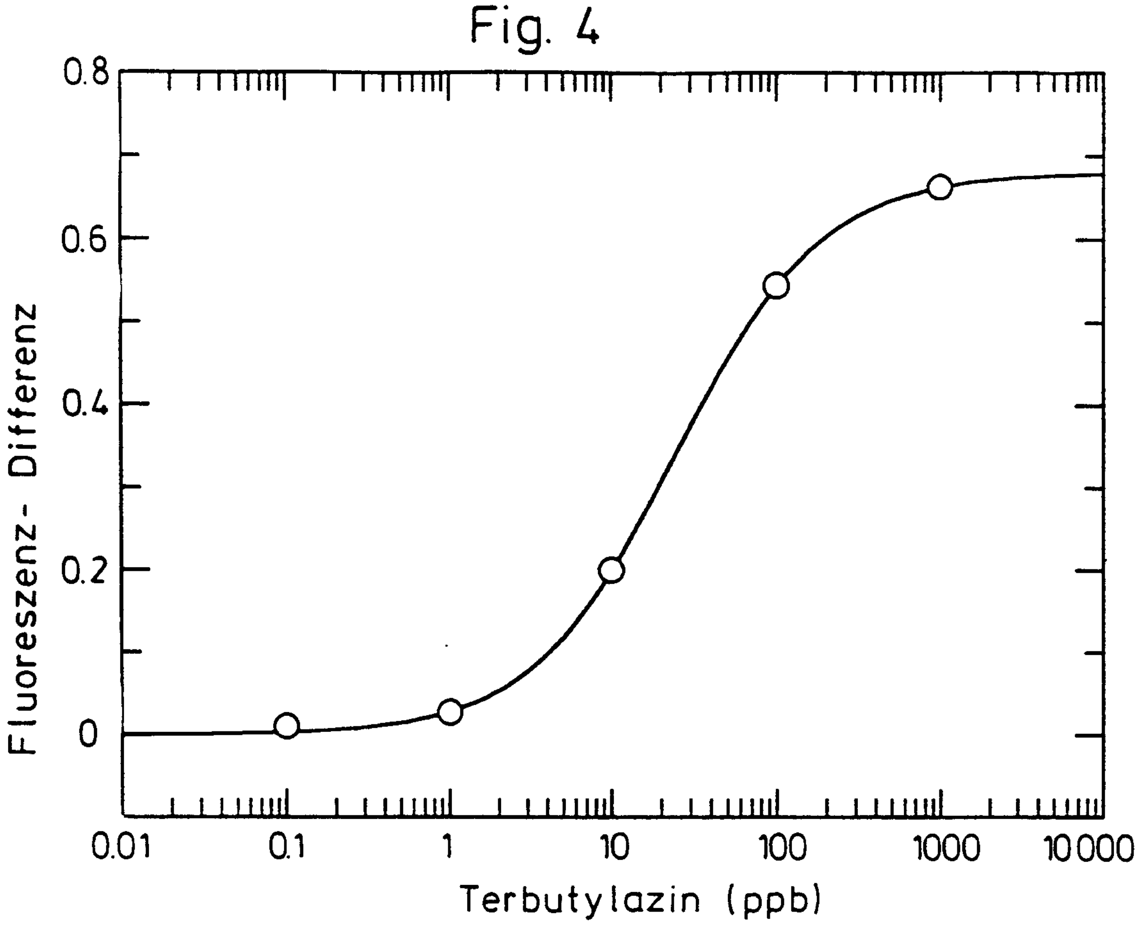
0.8
0.6
0.4
0.2
0
Fluoreszenz- Differenz
0.01
0.1
1
10
100
1000
10000
Terbutylazin (ppb)

Fig. 5

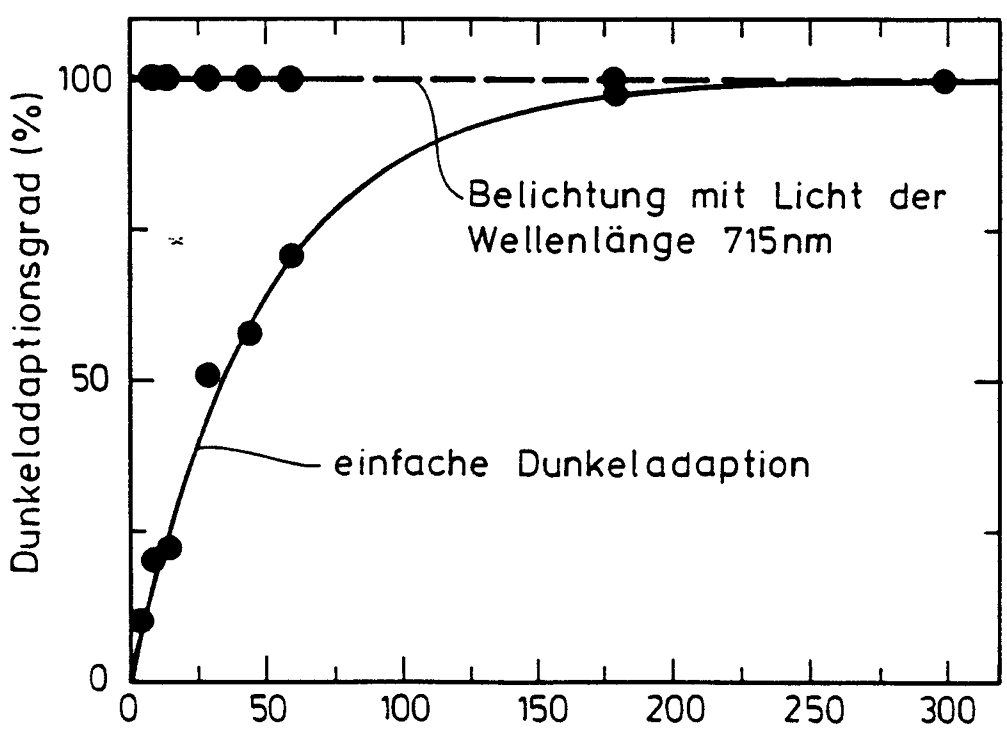
100
50
0
Dunkeladaptionsgrad (%)
Belichtung mit Licht der Wellenlänge 715nm
einfache Dunkeladaption
0
50
100
150
200
250
300
Dunkeladaptionszeit (s)

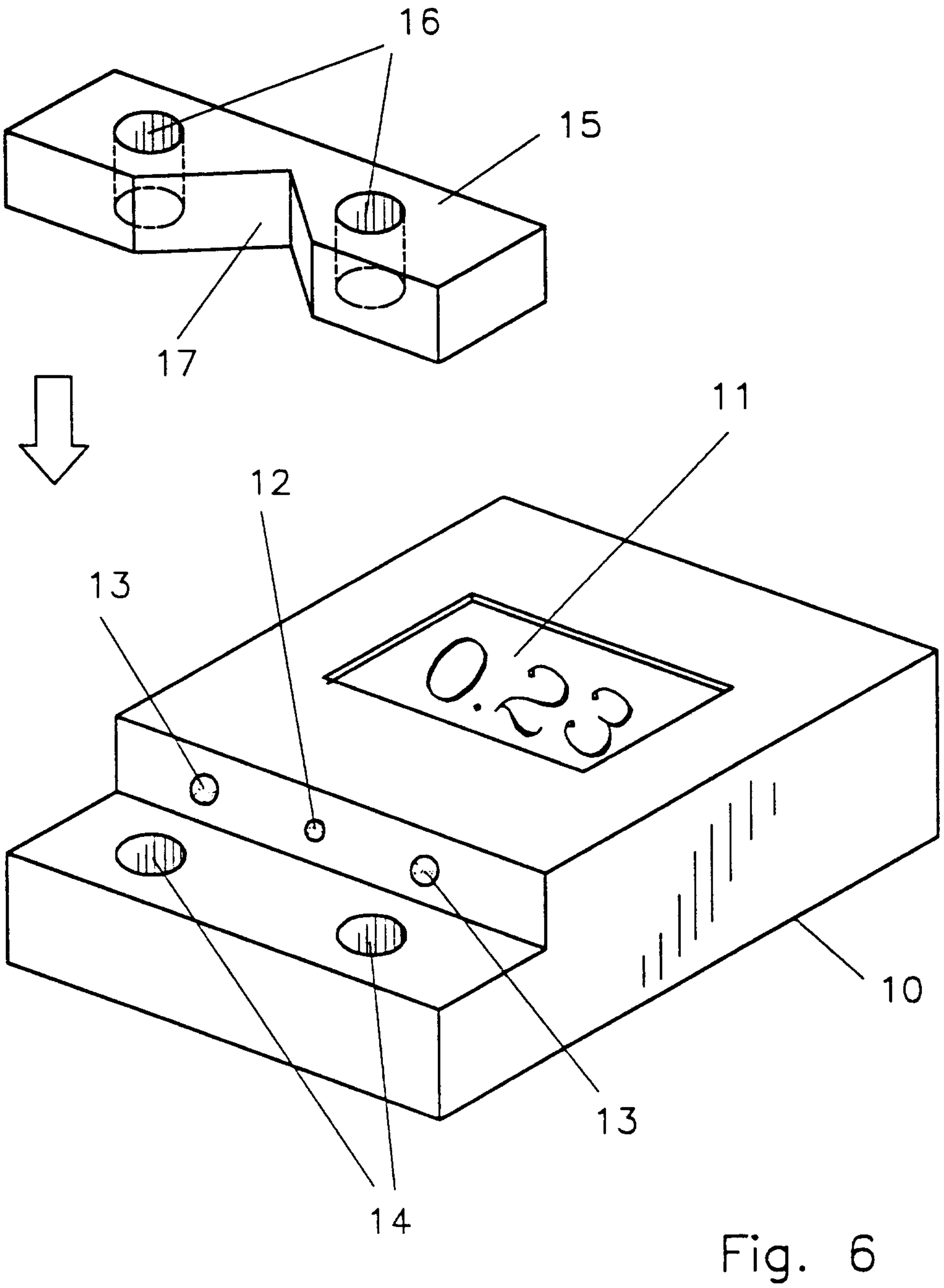


Fig. 6

Europäisches Patentamt

# EUROPÄISCHER RECHERCHENBERICHT

Nummer der Anmeldung
EP 94 11 3358

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br>Y | US-A-4 942 303 (KOLBER Z ET AL)<br>* Spalte 1, Zeilen 30-68; Spalte 3, Zeile 46 - Spalte 6, Zeile 42; Spalte 8, Zeilen 45-64; Spalte 11, Zeile 4 - Spalte 12, Zeile 51; Anspruch 9; Abbildungen 1,4,5 *<br>--- | 1,3,4<br>2 | G01N21/64 |
| Y<br>A | DE-A-34 12 023 (BUHLER E GMBH)<br>* das ganze Dokument *<br>--- | 2<br>1,5 | |
| A | DE-A-33 03 510 (GRABER P ET AL)<br>* das ganze Dokument *<br>--- | 1-3 | |
| A | EP-A-0 289 976 (KRAUSE H ET AL)<br>* Spalte 1, Zeile 35 - Spalte 2, Zeile 41; Spalte 7, Zeile 46 - Spalte 11, Zeile 1; Abbildungen 1-3, 5 *<br>--- | 1,2,5 | |
| X<br>Y<br>A | DE-A-41 10 548 (GBF GESELLSCHAFT FüR BIOTECHNOLOGISCHE FORSCHUNG GMBH)<br>* Seite 5, Zeile 16 - Zeile 46 *<br>--- | 5<br>6<br>1,2 | RECHERCHIERTE SACHGEBIETE (Int.Cl.6)<br>G01N |
| Y<br>A | CHEMICAL ABSTRACTS, vol. 119, no. 7, 16. August 1993, Columbus, Ohio, US; abstract no. 65532v, MATUSZCZYK G ET AL 'Critical evaluation of a screening test for detection of herbicides by inhibiting photosynthesis of isolated chloroplasts'<br>Seite 364-365 ;<br>* Zusammenfassung *<br>& MIKROCHIM. ACTA, Bd.110, Nr.1-3, 1993 Seiten 23 - 30<br>---<br>-/-- | 6<br>1,5 | |

Der vorliegende Recherchenbericht wurde für alle Patentansprüche erstellt

| Recherchenort | Abschlußdatum der Recherche | Prüfer |
|---|---|---|
| BERLIN | 10. Februar 1995 | Johnson, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 11 3358

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DATABASE ANALYTICAL ABSTRACTS<br>Royal Society of Chemistry, CAMBRIDGE GB<br>Accession No.: 54-01-H-00088<br>PURCELL M ET AL 'Immobilized plant thylakoid membranes as a biosensor for herbicides'<br>* Zusammenfassung *<br>& BIOTECHNOL. TECH.,<br>Bd.5, Nr.4, 1990<br>Seiten 363 - 368<br>----- | 1,5,6 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| BERLIN | 10. Februar 1995 | Johnson, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)